# EUROPEAN PATENT SPECIFICATION

(11) **EP 2 897 906 B1**
(45) Date of publication and mention of the grant of the patent: **05.07.2017**
(21) Application number: 12781446.5
(22) Date of filing: 20.09.2012
(51) Int. Cl.: C01G 28/00, A61K 33/36, A61K 8/02, A61Q 19/00, A61K 8/98, A61K 8/19, B82Y 30/00

(54) **PROCESS FOR BIO SYNTHESIS OF NANO ARSENIC TRIOXIDE AND ITS USE IN TREATMENT OF DISEASES INCLUDING CANCERS**
VERFAHREN ZUR SYNTHESE VON NANOARSENTRIOXID UND SEINE VERWENDUNG BEI DER BEHANDLUNG VON ERKRANKUNGEN EINSCHLIESSLICH KREBSERKRANKUNGEN
PROCÉDÉ DE BIO-SYNTHÈSE DE TRIOXYDE D'ARSENIC NANOMÉTRIQUE ET SON UTILISATION DANS LE TRAITEMENT DES MALADIES, Y COMPRIS LES CANCERS

(43) Date of publication of application: 29.07.2015
(73) Proprietor: Bendale, Yogesh Narayan, Pune 411052 (IN); Bendale, Vineeta Yogesh, Pune 411052 (IN)
(72) Inventor: Bendale, Yogesh Narayan, Pune 411052 (IN); Bendale, Vineeta Yogesh, Pune 411052 (IN)
(74) Representative: Simon, Josef
(86) International application number: PCT/IB2012/054992
(87) International publication number: WO 2014/045083

(56) References cited:
- DATABASE CAPLUS [Online] CHEMICAL ABSTRACTS SERVICE, COLUMBUS, OHIO, US; 22 January 2001 (2001-01-22), Becker, K. A.; Schultze, W.; Stranski, I. N.: "Condensation experiments with As2O3", XP002697203, Database accession no. 1963:407074
- PETER BALÁZ AND JÁN SEDLÁK: "Arsenic in Cancer Treatment: Challenges for Application of Realgar Nanoparticles (A Minireview)", TOXINS, vol. 2, 21 June 2010 (2010-06-21), pages 1568-1581, XP002697204, DOI: doi:10.3390/toxins2061568
- AHN RW, CHEN F, CHEN H, STERN ST, CLOGSTON JD, PATRI AK, RAJA MR, SWINDELL EP, PARIMI V, CRYNS VL, O'HALLORAN TV: "A novel nanoparticulate formulation of arsenic trioxide with enhanced therapeutic efficacy in a murine model of breast cancer.", CLIN. CANCER RES., vol. 16, no. 14, 2 June 2010 (2010-06-02), pages 3607-3617, DOI: 10.1158/1078-0432.CCR-10-0068
- CARRIE PRINTZ: "Arsenic nanoparticle holds promise in blocking aggressive breast cancer", CANCER, vol. 116, no. 24, 15 December 2010 (2010-12-15), page 5567, DOI: 10.1002/cncr.25816

## Description

### Field of Invention

The present invention is a novel process for production of nano arsenic trioxide with the aid of plant materials, buttermilk and goats urine. The process is environment friendly. The novel product obtained through the present inventive process is more bio available and therapeutically effective in the treatment of various diseases including but not limited to leukaemia, other types of malignant and benign tumours, rheumatism. The present inventive product also finds application in agriculture, horticulture, veterinary treatment and other fields where arsenic trioxide can be used as known to people skilled in the art.

### Background of Invention

Applicability of Arsenic minerals is known in traditional Chinese and Indian medicine since ancient times. In the 18^{th} Century Thomas Fowler compounded a potassium bicarbonate based solution of arsenic trioxide (As₂O₃) which came to be known as Fowler's solution. Pharmacology texts of the 18^{th} Century indicate the use of arsenical pastes for the treatment of variety of diseases including cancer [Karen H. Antman; Introduction: The history of arsenic trioxide in cancer therapy; The Oncologist 2001; 6(suppl 2):1-2]. In the 1990's it was reported by the Chinese investigators that herbal mixture containing arsenic trioxide could induce complete remission in patients suffering from acute promyelocytic leukaemia. [Dan Douer, Wendy Hu et. Al.; Arsenic trioxide (Trisenox) Therapy for Acute Promyelocytic Leukaemia in the setting of hematopoietic stem cell transplantation.; The Oncologist 2003; 8:132 - 140]

Arsenic widely exists in nature in its trivalent and pentavalent forms. Arsenic toxicity highly depends on its chemical form. In Indian and Chinese traditional medicines three forms of arsenic minerals i.e., Orpiment, Realgar and Arsenolite, are used alone and also in conjunction with other minerals for treatment of various diseases. The disposition of these arsenicals in the body depends on various key factors, including solubility, absorption, distribution, and excretion. Arsenic trioxide is the most bio available but it is highly toxic compared to Orpiment and Realger. Realgar is also a major component in bhasmas of Indian Ayurvedic medicine. Realger is used both for external as well as internal application. Arsenic trioxide, obtained after purification of Arsenolite, has short term toxicity due to which its therapeutic application is a major concern. To increase the therapeutic application of Realgar, nano particles of Realgar is prepared by cryogrinding with polyvinylpyrrolidone and Sodium Dodecyl Sulphate. (Jie Liu et. al., Mineral Arsenicals in Traditional Medicines:Orpiment, Realger, and Arsenolite, The Journal of Pharmacology and experimental therapeutics, Vol. 326, No.2, pg. 363-368,2008).

Prior art search indicates that arsenic trioxide is and has been used in the treatment of cancer. Peter Baláz and Ján Sedlák "Arsenic in Cancer Treatment: Challenges for Application of Realgar Nanoparticles (A Minireview)", TOXINS, vol. 2, 21 June 2010, pages 1568-1581 is a review dealing with arsenic in cancer treatment. The document discloses the treatment of some types of cancer with arsenic trioxide. AHN RW, CHEN F, CHEN H, STERN ST, CLOGSTON JD, PATRI AK, RAJA MR, SWINDELL EP, PARIMI V, CRYNS VL, O'HALLORAN TV "A novel nanoparticulate formulation of arsenic trioxide with enhanced therapeutic efficacy in a murine model of breast cancer", CLIN. CANCER RES., vol. 16, no. 14, 2 June 2010, pages 3607-3617 deals with a nanoparticulate formulation of arsenic trioxide and its therapeutic efficiency. EP2374463, EP2394702, EP 1562616, WO9924029, USPT 6,884,439 , USPT 6,855,339, USPT 6,982,096, USPT 6,723,351, USPT 6,720,011 are the results of prior art search indicating use of arsenic trioxide in therapy. DATABASE CAPLUS CHEMICAL ABSTRACTS SERVICE, COLUMBUS, OHIO, US, 22 January 2001, Becker, K. A., Schultze, W., Stranski, I. N. "Condensation experiments with As2O3" discloses a process for sublimation of arsenic trioxide. However the present invention is unique in terms of the process of preparation and the novel nano arsenic trioxide obtained through the present inventive process is generally of particle size ranging from 10nm to 1000nm. Further, the novel product is chemically more stable , bio available and safe due to less toxic nature for therapeutic administration in humans, animals and plants. The prior art process for production of arsenic trioxide that is used for therapeutic application involves purification of arsenic trioxide through chemical process. The present invention purifies arsenic trioxide through bio synthesis.

The present invention relates to the production of novel nano arsenic trioxide by a novel process, which involves purification, making it chemically more stable, and particle size reduction of arsenic trioxide, with plant materials, buttermilk and goat urine. The plant materials used in the present process are Dolichos biflorous, Momordica charantia, Zingiber officinale, Musa paradisica. The scope of the invention also include use of the active ingredients found in the herein mentioned plant materials, buttermilk, goat urine or other plant, organic or inorganic materials, containing cellulose, with similar characteristics and chemical properties, as known to the people skilled in the art, for the purpose of inventive process and production of the novel product.

Buttermilk is a fermented dairy product obtained from cow's milk. The tartness of buttermilk is due to the presence of lactic acid. The pH level of buttermilk is generally noted to be 4.41 to 4.84. Butter milk can be made through traditional process, by culturing and also through acidification process of milk. One phase of the invention involves purification of arsenic trioxide with the aid of buttermilk. Use of buttermilk is one embodiment of this invention and it is possible to derive the same results if buttermilk is substituted with any other fermented form of milk either obtained through traditional methods or cultured. The substitution of fermented milk obtained through traditional methods or cultured with that of chemically produced or induced active ingredients with same effect known to people skilled in the art cannot be ruled out.

Goat and Cow urine have been an ingredient in Indian Ayurvedic medicine and other traditional Indian Medicine. Goat Urine is used in treatment of tuberculosis by tribals in Western ghats, India [P. Padmanabhan & K.A. Sujana, 2008, Animal products in traditional medicine from Attapady hills of Western Ghats, Indian Journal of Traditional Medicine, Vol.7(2), pg.326-329]. However, the use of the goat urine as in the present process is not traditional knowledge or anticipated.

The medicinal values of plant materials belonging to genus Zingiber and family Zingiberaceae is well known in traditional medicines. Zingiber Officinale is plant species of genus Zingiber. The rhizome of Zingiber Officinale, Ginger, is one of the most widely used species of ginger family Zingiberaceae and is known for its medicinal use in traditional Chinese and Indian medicine. Ginger has many active ingredients such as Sesquiterpene hydrocarbons predominantly Zingiberene, active gingerols that can be converted to shogaols, Zingerone, Paradol. 6-Gingerol and 6- shogaols have shown pharmacological activities including anti pyretic, analgesic and in treatment of chemotherapy induced nausea (Monograph, Alternative Medicine Review, Volume 8, No. 3, 2003, pg. 331 -335). However, use of ginger or extract of plant material of family of Zingiber Officinale for particle size reduction of metalloid is not known. The present inventive process also involve particle size reduction of arsenic trioxide to nano particles with aid of ginger.

Momordica charantia (Bitter Melon) is a tropical vegetable of family Cucurbitaceae. It is a flowering vine with known medicinal properties. Its fruits contain glycosides, saponins, alkaloids, reducing sugars, resins, phenolic constituents, fixed oils and free acids. Chemical constituents in Momordica Charantia are Alkaloids, charantin, charine, cryptoxanthin, cucurbitins, cucurbitacins, cucurbitanes, cycloartenols, diosgenin, elaeostearic acids,erythrodiol, galacturonic acids, gentisic acid, goyaglycosides, goyasaponins, guanylate cyclise inhibitors, gypsogenin, hydroxytryptamines, karounidiols, lanosterol, lauric acid, linoleic acid, linolenic acid, momorcharasides, momorcharins, momordenol, momordicilin, momordicins, momordicinin, momordicosides, momordin, momordolo, multiflorenol, myristic acid, nerolidol, oleanolic acid, oleic acid, oxalic, acid, pentadecans, peptides, petroselinic acid, polypeptides, proteins, ribosome-inactivating proteins, rosmarinic acid, rubixanthin, spinasterol, steroidal glycosides, stigmasta-diols, stigmasterol, taraxerol, trehalose, trypsin inhibitors, uracil, vacine, v-insulin, verbascoside, vicine, zeatin, zeatin riboside, zeaxanthin, zeinoxanthin Amino acids-aspartic acid, serine, glutamic acid, thscinne, alanine, g-amino butyric acid and pipecolic acid, ascorbigen, b-sitosterol-d-glucoside, citrulline,elasterol, flavochrome, lutein, lycopene, pipecolic acid.

Musa paradisica is a herbaceous plant of the genus Musa. The older scientific name Musa paradisica is seldom used due to the advent of hybrid varieties. Presently the species are referred to as Musa acuminata or Musa balbisiana or triploid hybrids. The fruit as well as the plant materials have medicinal value. In the present invention the extract of the plant material is used.

Dolichos biflorous is commonly used as an astringent, diuretic and tonic. The enzymes found in Dolichos biflorous have been identified as Urease, Allantoinase, Ribonuclease, Nicotinamide deaminase, b-n-acetylglucosaminidase, a and b galactosidase, a-mannosidase and b glucosidase.

### Summary of Invention

To increase the therapeutic applicability and bio availability of arsenic trioxide, the present invention purifies, through bio purification, arsenic trioxide available in the market by the process of boiling, trituration, and heating, with plant materials, buttermilk and goats urine. In short, the arsenic trioxide in its crude form as available in the market is put through process of purification by boiling with buttermilk, Goat urine and Dolichos biflorous in consecutive steps. Thus purified arsenic trioxide is put through particle size reduction by process of trituration with extract of plant material of taxonomical genus Momordica and extract of rizhome of taxonomical genus Zingiber. The product obtained from trituration is then heated with extract of plant material of genus Musa. The process of heating is carried out in a container with closed lid so as to collect the sublimed product. The product collected from the inner side of the lid of the container is the novel nano arsenic trioxide with high therapeutic value and solubility. Further, bioavailability and therapeutic applicability of the novel product, nano arsenic trioxide, obtained through this inventive process is higher due to its less toxic nature and particle size.

### Detailed Description

Arsenic trioxide, crude powder form, available in the market, is bio purified with the aid of butter milk, Goat urine, and aqueous extract of Dolichos biflorous in various steps followed by trituration and heating as detailed below:
Step 1: Arsenic trioxide in coarse powder form is submerged into an earthen vessel containing boiling butter milk. The arsenic trioxide is hung to submerge into the boiling butter milk without touching sides of the earthen vessel. This process is carried out for seven hours. At all times the arsenic trioxide should be submerged during the process of boiling. Hence, buttermilk will have to be added repeatedly during the process of boiling to maintain arsenic trioxide in submerged condition throughout the period.
Step 2: After boiling arsenic trioxide with butter milk in accordance with step 1, the same is hung and submerged into boiling goat urine contained in an earthen vessel, without touching the sides of the container. This process is carried out for seven hours. During this step too arsenic trioxide is to be retained submerged through the whole period of boiling with goat urine and hence it would be required to add goat urine during the process to maintain the arsenic trioxide in submerged condition.
Step 3: After boiling arsenic trioxide with goat urine in accordance with step 2, the same is then hung and submerged, into boiling aqueous extract of Dolichos biflorous contained in an earthen vessel, without touching the sides of the container. This process is carried out for 7 hours. Similar to the earlier steps, addition of aqueous extract of Dolichos biflorous repeatedly is required throughout the period of boiling to maintain the arsenic trioxide completely submerged.

The time of seven hours for the process continuation provided in step 1 to 3 cannot be considered as limiting. In the process arsenic trioxide can be hung using a cotton string or with materials of similar characteristic. The vessel used can be an earthen container or any vessel with similar characteristics that may be known to persons skilled in the art. Product is yellowish white powder. Biological variation that may occur in goat urine, buttermilk, and the extract of plant materials is anticipated in the present invention. The scope of the present invention also anticipates the use of buttermilk, goat urine and extract of dolichos biflorous in interchangable order, as in similar result is possible if the Arsenic Trioxide is put through step 1, 2 or 3 in different orders where by step 3 or step 2 can be performed as step 1 or vice versa.

Step 4: The product of Step 3 is triturated with extract of Momordica charantia for 7 hours. The time for trituration cannot be considered limiting. The process of trituration is repeated thrice consecutively, i.e., once the product of trituration is completely dry then the next trituration should be commenced with. Generally it takes 7 hours for the mixture to dry out but 7 hours cannot be considered limiting, as the number of hours depends upon the quantity of the material.

Step 5: The product of Step 4 is triturated with extract of ginger, rhizome of Zingiber officinale, for 7 hours. The time for trituration cannot be considered limiting. Similar to Step 4, the trituration is carried out thrice consecutively whereby the dry product obtained after trituration is further triturated with extract of rhizome of Zinziber Officianale until dry.

The time provided for trituration i.e., seven hours cannot be considered limiting and the desired result can be obtained if the trituration is carried out for less or more hours. The particle size of the product would vary with the number of hours of trituration. The possibility of obtaining the desired result if the trituration is carried out with a mixture of the plant materials or its active ingredients, specified in steps 4 and 5, or other organic or inorganic materials with similar characteristics, is within the scope of this invention. The resultant product is generally yellowish white in colour.

Step 6: The product of Step 5 is heated with extract of the stem of Musa paradisica in a closed earthen container with removable lid, volume ratio of the product and extract of the stem of Musa paradisica is preferably 1:10. The heating is gradual and carried out for 12 hours. The heating duration cannot be considered limiting. The sublimated product on the inner side of the lid of the container is the final product. The final product is generally white in colour with mild yellow tinge. The maximum temperature to which the present process is subjected to is approximately 500 degree centigrade. However, the desirable result can be obtained at below or above 500 degree centigrade and hence the temperature of heating cannot be considered as limiting. The temperature at which the product is obtained would vary with the quantity of the crude arsenic trioxide and other ingredients.

The container or apparatus for carrying out the present process is an earthen vessel with a removable lid or any other vessel, which is known to the people skilled in the art to bring about similar results.

The ingredients in the steps mentioned above are one embodiment of the invention. The scope of the invention also include active ingredients of the plant and other materials used in the process, organic as well as inorganic, or other plant materials and organic or inorganic materials with similar characteristics or chemical properties, containing cellulose, that are known to people skilled in the art to bring desired effects on substitution of buttermilk, Goat Urine, Dolichos biflorous, Momordica Charantia, Ginger, Musa Paradisica or other aids in this novel process for production of nano arsenic trioxide.

The administration of this chemically more stable , novel nano arsenic trioxide obtained through this inventive process can be done as is or in combination with other minerals, metals, chemical elements or compounds. Both in vitro and in vivo administration is possible with this novel nano arsenic trioxide. The oral administration of the product can be in conjunction with honey or water or any other suitable carrier. The dosage of administration can range from 0.01 mg per kg body weight to 10 mg per kg body weight. It can be administered on daily basis depending on the nature of the disease. In case of cancer the period of administration can be until complete remission or reduction of tumour as the case may be.

The dosage form can be in powder, tablet, capsule, caplet, effervescent, fluid, gelatinous, granules or in any other palatable and administrable form.

The nano arsenic trioxide obtained through this process, generally of size varying from 10nm to 1000nm, can also be used in nutraceutical, herbal and mineral composition apart from pharmaceutical or other therapeutic composition.

The product of the present inventive process is also effective in treatment of cancers which are malignant and benign, haematological malignancies such as Promyelocytic Leukemia, other types of Leukemias, Lymphomas, solid tumours like Lung Cancers, Liver cancers etc. The application of this Nano Arsenic Trioxide is not only limited to various cancers but also includes other degenerative and metabolic disorders of vital organs such as lungs, liver, brain, kidneys etc. This has wide application in Neurological cancer disorders and also in multiple infective disorders especially but not limiting to antibiotics or anti microbial resistant infections such as Tuberculosis .This novel arsenic trioxide has got specific action on various enzymatic and hormonal activities in the body for treatment of diseases, including but not limited to, Diabetes. It has got specific action on bone developments in general and at epiphyseal level including prevention and cure of bone and other types of cancers. Use of the present novel product in the treatment of animals, plants, herbs or other flora and industrial application in nutraceutical, cosmetic, herbal, mineral and other composition is anticipated.

The following are a few examples. However the examples provided hereunder must not be considered limiting as to the scope, working or use of the present invention:

Examples of Formulation:

**[Table 1]**

| Sr.No. | Arsenic Trioxide dose in mg | Weight of lactose monohydrate in mg | Magnesium Stearate in mg | Total weight in mg/ capsule | Actual weight of powder to be filled in mg/ capsule |
|---|---|---|---|---|---|
| 1 | 6 | 57.00 | 0.5 | 63.50 | 64* |
| 2 | 12 | 56.00 | 0.5 | 68.50 | 69* |

| | | | | | |
|---|---|---|---|---|---|
| * Quantity made up with lactose monohydrate | | | | | |

The quantity of yield of the bio purified nano arsenic trioxide depends upon the quantity of the crude form of Arsenic Trioxide used in the inventive process. For example, if 100gm of crude Arsenic Trioxide is used in the present inventive process, the quantity of yield of the bio purified therapeutically effective Arsenic Trioxide will be in the range of 75-80 gms. Further, the quantity also varies depending on the quality of the crude arsenic trioxide used in the process.

## Claims

1. The process comprising of, submergence and boiling of crude powder form of arsenic trioxide in buttermilk, Goat urine, and in aqueous extract of dolichos biflorus, followed by trituration of the arsenic trioxide obtained after the aforesaid boiling with the extract of plant material Momordica charantia until dry and further trituration of the said dry product with extract of ginger until dry, subsequent heating of the product obtained after the trituration with the extract of Musa paradisica in an apparatus with lid, and collection of sublimed product, nano arsenic trioxide, from the inner side of the lid.

2. The product obtained through process claimed in claim 1.

3. The process claimed in claim 1 carried out by substitution of buttermilk, Goat urine and extract of dolichos biflorous, with their active ingredients, or other organic or inorganic materials, including plants containing cellulose, with similar characteristics and chemical properties as that of buttermilk, Goat urine and extract of dolichos biflorous.

4. The process claimed in claim 1 carried out by substitution of Momordica charantia with extract of plant materials of taxonomical genus Momordica, its active ingredients or other plant materials, other organic or inorganic materials, containing cellulose, with similar characteristics and chemical properties as that of Momordica Charantia.

5. The process claimed in claim 1 carried out by substituting ginger with rhizome of plant materials of taxonomical family Zingiberaceae or other tuberous plants, combination thereof, its active ingredients, or plant material, other organic or inorganic materials, containing cellulose, with similar characteristics and chemical properties as that of Ginger.

6. The process claimed in claim 1, wherein Musa Paradisica is substituted with plant materials of taxonomical Genus Musa or its active ingredients or other plant materials, organic or inorganic materials, containing cellulose, with similar characteristics and chemical properties as that of Musa paradisica.

7. Nano arsenic trioxide obtained through the process claimed in any of claims 1, 3-6 in any palatable or administrable form, for use in the treatment of cancer in a human or an animal in a dosage ranging from 0. 01 mg/kg to 10mg/kg body weight for therapy.

8. The use of the nano arsenic trioxide obtained through the process claimed in any of claims 1, 3-6 in a suitable dosage form for treatment of herbs, plants and other flora.

9. The use of the nano arsenic trioxide obtained through the process claimed in claim 1, in nano technology, material science, agriculture, bio technology and other fields of applicability.

10. The use of the nano arsenic trioxide obtained through process claimed in any of claims 1, 3-6 in pharmaceutical composition, other therapeutic composition, nutraceutical, herbal, cosmetic or mineral compositions.

11. Dosage form of product obtained through process claimed in any of claims 1, 3 to 6 in any palatable and administrable form.

## Patentansprüche

1. Verfahren umfassend ein Eintauchen und Kochen einer unverarbeiteten Pulverform von Arsentrioxid in Buttermilch, Ziegenurin und in einen/einem wässrigen Extrakt von *Dolichos biflorus,* gefolgt von einem Zerreiben des nach dem vorgenannten Kochen erhaltenen Arsontrioxids mit dem Extrakt des Pflanzenmaterials *Momordica charantia* bis es trocken ist und weiteres Zerreiben des genannten trockenen Produkts mit einem Ingwerextrakt bis es trocken ist, anschließendes Erhitzen des nach dem Zerreiben erhaltenen Produkts mit dem Exakt von *Musa paradisica* in einem Apparat mit Deckel und Sammeln des sublimierten Produkts, Nanoarsentrioxid, von der Innenseite des Deckels.

2. Produkt erhalten nach dem Verfahren gemäß Anspruch 1.

3. Verfahren nach Anspruch 1, ausgeführt durch Ersatz von Buttermilch, Ziegenurin und dem Extrakt von *Dolichos biflorus* durch ihre aktiven Inhaltsstoffe oder andere organische oder anorganische Materialien, einschließlich Zellulose enthaltenden Pflanzen, mit ähnlichen Charakteristika und chemischen Eigenschaften wie denen von Buttermilch, Ziegenurin und dem Extrakt von *Dolichos biflorus.*

4. Verfahren nach Anspruch 1, ausgeführt durch Ersatz von *Momordica charantia* durch einen Extrakt von Pflanzenmaterialien des taxonomischen Genus *Momordica,* dessen aktive Inhaltsstoffe oder andere Pflanzenmaterialien, andere organische oder anorganische Materialien, enthaltend Zellulose, mit ähnlichen Charakteristika und chemischen Eigenschaften wie denen von *Momordica charantia.*

5. Verfahren nach Anspruch 1, ausgeführt durch Ersetzen von Ingwer durch ein Rhizom von Pflanzenmaterialien der taxonomischen Familie *Zingiberaceae* oder andere knollige Pflanzen, eine Kombination davon, deren aktive Inhaltsstoffe, oder Pflanzenmaterial, andere organische oder anorganische Materialien, enthaltend Zellulose, mit ähnlichen Charakteristika und chemischen Eigenschaften wie denen von Ingwer.

6. Verfahren nach Anspruch 1, wobei *Musa paradisica* ersetzt wird durch Pflanzenmaterialien des taxonomischen Genus *Musa* oder deren aktive Inhaltsstoffe oder andere Pflanzenmaterialien, organische oder anorganische Materialien, enthaltend Zellulose, mit ähnlichen Charakteristika und chemischen Eigenschaften wie denen von *Musa paradisica.*

7. Nanoarsentrioxid erhalten durch das Verfahren nach einem der Ansprüche 1, 3-6 in jeder genießbaren oder verabreichbaren Form, zur Verwendung bei der Behandlung von Krebs in einem Menschen oder einem Tier in einer von 0,01 mg/kg bis 10 mg/kg Körpergewicht reichenden Dosierung zur Therapie.

8. Verwendung des nach dem Verfahren gemäß einem der Ansprüche 1, 3-6 erhaltenen Nanoarsentrioxids in einer geeigneten Darreichungsform zur Behandlung von Kräutern, Pflanzen und anderer Flora.

9. Verwendung des nach dem Verfahren nach Anspruch 1 erhaltenen Nanoarsentrioxids in der Nanotechnologie, Materialwissenschaft, Landwirtschaft, Biotechnologie oder anderen Bereichen der Anwendbarkeit.

10. Verwendung des nach einem Verfahren gemäß einem der Ansprüche 1, 3-6 erhaltenen Nanoarsentrioxids in einer pharmazeutischen Zusammensetzung, einer anderen therapeutischen Zusammensetzung, nutrazeutischen, pflanzlichen, kosmetischen oder mineralischen Zusammensetzungen.

11. Darreichungsform des nach einem Verfahren gemäß einem der Ansprüche 1, 3-6 erhaltenen Produkts in jeder genießbaren und verabreichbaren Form.

## Revendications

1. Procédé constitué de la submersion et l'amenée à ébullition d'une forme de poudre brute de trioxyde d'arsenic dans du babeurre, de l'urine de chèvre et dans un extrait aqueux de Dolichos biflorus, suivies de la trituration du trioxyde d'arsenic obtenu après l'amenée à ébullition susmentionnée avec l'extrait de matière végétale Momordica charantia jusqu'à séchage et trituration supplémentaire dudit produit sec avec un extrait de gingembre jusqu'à séchage, chauffage subséquent du produit obtenu après la trituration avec l'extrait de Musa paradisica dans un appareil avec couvercle et recueil du produit sublimé, le trioxyde d'arsenic nanométrique, du côté interne du couvercle.

2. Produit obtenu par le procédé selon la revendication 1.

3. Procédé selon la revendication 1 effectué par substitution de babeurre, d'urine de chèvre et d'extrait de Dolichos biflorus par leurs ingrédients actifs, ou autres matières organiques ou inorganiques, incluant des plantes contenant de la cellulose, avec des caractéristiques et propriétés chimiques similaires à celles du babeurre, de l'urine de chèvre et de l'extrait de Dolichos biflorus.

4. Procédé selon la revendication 1 effectué par substitution de Momordica charantia par un extrait de matières végétales du genre taxinomique Momordica, ses ingrédients actifs ou autres matières végétales, autres matières organiques ou inorganiques, contenant de la cellulose, avec des caractéristiques et propriétés chimiques similaires à celles de Momordica charantia.

5. Procédé selon la revendication 1 effectué en substituant au gingembre un rhizome de matières végétales de la famille taxinomique Zingiberaceae ou d'autres plantes tubéreuses, une combinaison de celles-ci, ses ingrédients actifs, ou matière végétale, autres matières organiques ou inorganiques, contenant de la cellulose, avec des caractéristiques et propriétés chimiques similaires à celles du gingembre.

6. Procédé selon la revendication 1, dans lequel Musa paradisica est substituée par des matières végétales du genre taxinomique Musa ou ses ingrédients actifs ou autres matières végétales, matières organiques ou inorganiques, contenant de la cellulose, avec des caractéristiques et propriétés chimiques similaires à celles de Musa paradisica.

7. Trioxyde d'arsenic nanométrique obtenu par le procédé selon l'une quelconque des revendications 1, 3 à 6 sous toute forme palatable ou administrable, destiné à être utilisé dans le traitement du cancer chez l'homme ou l'animal dans un dosage allant de 0,01 mg/kg à 10 mg/kg de poids corporel pour thérapie.

8. Utilisation du trioxyde d'arsenic nanométrique obtenu par le procédé selon l'une quelconque des revendications 1, 3 à 6 sous une forme de dosage appropriée pour le traitement d'herbes, de plantes et autre flore.

9. Utilisation du trioxyde d'arsenic nanométrique obtenu par le procédé selon la revendication 1 en nanotechnologie, sciences de la matière, agriculture, biotechnologie et dans d'autres domaines d'applicabilité.

10. Utilisation du trioxyde d'arsenic nanométrique obtenu par le procédé selon l'une quelconque des revendications 1, 3 à 6 dans une composition pharmaceutique, autre composition thérapeutique, des compositions nutraceutiques, à base de plantes, cosmétiques ou minérales.

11. Forme de dosage du produit obtenu par le procédé selon l'une quelconque des revendications 1, 3 à 6 sous toute forme palatable et administrable.
